# EUROPÄISCHE PATENTSCHRIFT

(11) **EP 3 349 691 B1**
(45) Veröffentlichungstag und Bekanntmachung des Hinweises auf die Patenterteilung: **05.01.2022**
(21) Anmeldenummer: 16766903.5
(22) Anmeldetag: 12.09.2016
(51) Int. Cl.: A61F 2/16

(54) **INTRAOKULARLINSENZUFÜHRSYSTEM MIT EINEM HEIZELEMENT**
INTRAOCULAR LENSE SUPPLY SYSTEM COMPRISING A HEATING ELEMENT
SYSTÈME D'APPORT DE LENTILLE INTRAOCULAIRE POURVU D'UN ÉLÉMENT CHAUFFANT

(30) Priorität: 14.09.2015 DE 102015217495
(43) Veröffentlichungstag der Anmeldung: 25.07.2018
(73) Patentinhaber: Carl Zeiss Meditec AG, 07745 Jena (DE)
(72) Erfinder: MÜLLER, Marco, 10437 Berlin (DE)
(74) Vertreter: Carl Zeiss AG - Patentabteilung
(86) Internationale Anmeldenummer: PCT/EP2016/071417
(87) Internationale Veröffentlichungsnummer: WO 2017/046028

(56) Entgegenhaltungen:
- WO-A1-2015/161837
- US-A1- 2008 097 460
- US-A1- 2011 264 103

## Beschreibung

Die Erfindung betrifft ein Intraokularlinsenzuführsystem.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der ein dünnes Rohr in die erkrankte Linse eingeführt und mit Ultraschall zu Schwingungen angeregt wird. Durch die Schwingungen kommt es zu einem Aufbrechen bzw. einer Emulsifikation der harten Linse in kleine Partikel, welche durch das Rohr mittels einer Pumpe abgesaugt werden können. Dabei wird ein Spülfluid (Irrigationsfluid) zugeführt, wobei das Absaugen der Partikel und des Fluides durch eine an das Rohr angeschlossene Aspirationsleitung erfolgt. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Das Einsetzen der künstlichen Linse, welche als Intraokularlinse bezeichnet wird, erfolgt mittels eines Intraokularlinsenzuführsystems. Das Intraokularlinsenzuführsystem weist eine Kanüle auf, durch welche die Intraokularlinse mittels eines Kolbens vorwärts bewegt und in den Kapselsack des Patienten eingeschoben wird. Damit die Verletzung der Hornhaut so klein wie möglich ausfallen kann, ist der Durchmesser der Kanüle mit z.B. 1,6 mm sehr klein. Ein derart kleiner Durchmesser erfordert es jedoch, dass die Intraokularlinse in einem eingerollten Zustand durch die Kanüle befördert wird. Um die Intraokularlinse einrollen zu können, besteht die Intraokularlinse aus einem weichen Werkstoff, welcher üblicherweise ein Kunststoff ist. Die Werkstoffauswahl beeinflusst zum einen die Fähigkeit der Intraokularlinse zum Einrollen in eine enge Kanüle, zum anderen beeinflusst er die Elastizität der Intraokularlinse während der Vorwärtsbewegung in der Kanüle. Es ist bekannt, dass diese Eigenschaften deutlich von der Temperatur des Werkstoffes der Intraokularlinse abhängen. Eine zu niedrige Temperatur kann zu Rissen in der Intraokularlinse während der Vorwärtsbewegung in der Kanüle führen, wohingegen eine zu hohe Temperatur eine bleibende Verformung der Intraokularlinse bewirken kann.

Es ist aus der EP 2 083 753 B1 ein heizbares Intraokularlinsenzuführsystem bekannt, mit dem sich für die Intraokularlinse eine gewünschte Temperatur erreichen lässt. Dazu ist es erforderlich, dass elektrische Verbindungen und eine Energiequelle vorgesehen sind. Die elektrischen Verbindungen können aufgrund von Kontaktproblemen versagen, sodass eine Heizung vollständig ausfällt. Ist die Energiequelle eine Batterie, kann diese das Gewicht des Intraokularlinsenzuführsystems signifikant erhöhen. Zudem ist die Einsatzdauer von Batterien begrenzt, sodass es passieren kann, dass während einer Operation die Energiezufuhr nachlässt oder nicht mehr verfügbar ist. Wenn eine Energiequelle außerhalb des Intraokularlinsenzuführsystems vorgesehen ist, sind elektrische Verbindungskabel erforderlich, welche die Handhabbarkeit des Intraokularlinsenzuführsystems negativ beeinflussen. Um die gewünschte Temperatur für die Intraokularlinse zu erzielen, ist eine Temperatureinstellung und eventuell eine Sensorik für das Erreichen der Solltemperatur erforderlich. Dies erhöht den technischen Aufwand für ein Intraokularlinsenzuführsystem beträchtlich. Falls die gewünschte Temperatur regelbar sein soll, erhöht dies den technischen Aufwand noch mehr.

Ein Intraokularlinsenzuführsystem zum Einsetzen einer Intraokularlinse in ein Auge gemäß dem Oberbegriff des Anspruchs 1 ist in US 2011/0264103 A1 beschrieben.

Es ist eine Aufgabe der Erfindung, ein heizbares Intraokularlinsenzuführsystem zu schaffen, welches nur mit einem geringen technischen Aufwand verbunden ist, mit einer minimalen Energiezufuhr auskommt, keine elektrischen Kontaktprobleme aufweist und so gut handhabbar ist wie ein nicht heizbares Intraokularlinsenzuführsystem.

Die Aufgabe wird durch den Gegenstand des unabhängigen Patentanspruchs 1 gelöst. Vorteilhafte Weiterbildungen der Erfindung sind Gegenstand der Unteransprüche.

Das erfindungsgemäße Intraokularlinsenzuführsystem zum Einsetzen einer Intraokularlinse in ein Auge weist auf:
- ein Gehäuse,
- eine in das Gehäuse einsetzbare Kassette zur Aufnahme einer Intraokularlinse,
- ein Heizelement,
   dadurch gekennzeichnet, dass das Heizelement einen Latentwärmespeicher und einen Keimbildner aufweist,
   und das Intraokularlinsenzuführsystem ein Druckelement aufweist, welches mit der Kassette oder dem Gehäuse gekoppelt ist und so verlagerbar ist, dass das Druckelement bei in das Gehäuse eingesetzter Kassette auf den Keimbildner eine Druckkraft ausübt, sodass vom Heizelement Wärmeenergie an die Kassette zugeführt wird.

Ein Latentwärmespeicher kann thermische Energie verborgen, verlustarm, mit vielen Wiederholzyklen und über lange Zeit speichern. Bei einem Latentwärmespeicher ist die latente Schmelzwärme, Lösungswärme oder Absorptionswärme wesentlich größer als die Wärme, die er aufgrund seiner normalen spezifischen Wärmekapazität speichern könnte. Bei einem Latentwärmespeicher wird die Enthalpie thermodynamischer Zustandsänderungen genutzt. Das "Aufladen" eines Latentwärmespeichers kann zum Beispiel durch das Schmelzen bestimmter Werkstoffe erfolgen, wobei diese Werkstoffe sehr viel Wärmeenergie (Schmelzwärme) aufnehmen. Das "Entladen" erfolgt dann nach einer Aktivierung als Erstarren, wobei der Latentwärmespeicher die zuvor aufgenommene große Wärmemenge als Erstarrungswärme wieder an seine Umgebung abgibt. Eine Unterkühlung der Schmelze ist jedoch unerwünscht, sodass dem Latentwärmespeicher gemäß der Erfindung Keimbildner zugesetzt sind, die eine Kristallisation kurz unterhalb der Schmelztemperatur bewirken.

Der Latentwärmespeicher kann ein Salzhydrat MₙH₂O sein, wie z. B. Na₂SO₄ · 10 H₂O (32,5 °C) oder NaCl · Na₂SO₄ · 10 H₂O (32,4 °C). Der Latentwärmespeicher kann auch eine übersättigte Lösung aus Natrium-Acetat Na(CH₃COO) oder NaOAc (58 °C) sein.

Gemäß der Erfindung weist das Intraokularlinsenzuführsystem, welches bevorzugt ein Injektor ist, ein Druckelement auf, welches mit der Kassette oder dem Gehäuse gekoppelt ist und so verlagerbar ist, dass das Druckelement bei in das Gehäuse eingesetzter Kassette auf den Keimbildner eine Kraft ausübt. Bei dem Druckelement ist die Kraft auf den Keimbildner so zu dimensionieren, dass eine Kristallisation im Latentwärmespeicher ausgelöst und dadurch die latente Wärmeenergie freigegeben wird. Dies kann durch eine Kraft von zum Beispiel 30 bis 50 N auf den Keimbildner erreicht werden.

Das Druckelement kann vorgespannt sein, sodass ein Bediener des Intraokularlinsenzuführsystems durch Freigabe der Vorspannung die Druckkraft freigibt, welche dann auf den Keimbildner wirken kann. Andererseits kann das Druckelement durch ein vom Bediener erfolgtes Verlagern, zum Beispiel durch Translation oder Rotation, eine statische Druckkraft auf den Keimbildner ausüben. Es ist dabei möglich, dass die Druckkraft auf den Keimbilder zunehmend einwirkt, während die Kassette in das Intraokularlinsenzuführsystem eingesetzt wird. Die Kraftausübung erfolgt dann zwangsweise während des Einsetzvorganges der Kassette in das Intraokularlinsenzuführsystem. Jedoch ist es ebenso möglich, dass bei bereits in das Intraokularlinsenzuführsystem eingesetzter Kassette, also bei einem sogenannten vorgeladenen Intraokularlinsenzuführsystem, vom Druckelement die Druckkraft auf den Keimbilder erstmals ausgeübt wird, wenn dem Bediener der Zeitpunkt dafür als geeignet erscheint. Dieser Zeitpunkt kann von der Zeitdauer abhängen, in der die Kassette bzw. die darin aufgenommene Intraokularlinse nach Auslösen der Kristallisation eine gewünschte Temperatur erreicht.

Gemäß der Erfindung ist das Druckelement verlagerbar. Damit ist es möglich, eine Position des Druckelementes und somit eine relative Lage zwischen dem Druckelement und der Kassette oder dem Druckelement und dem Gehäuse zu verändern. Dies bedeutet, dass auch die Druckkraft, welche das Druckelement auf den Keimbildner ausüben kann, verändert werden kann. Wenn die erforderliche Druckkraft zur Auslösung der Kristallisation des Keimbildners nicht ausreichen sollte, zum Beispiel aufgrund von Fertigungsungenauigkeiten einer Kassette, kann durch die Verlagerung des Druckelementes die Kraft auf einfache Weise erhöht werden. Die Verlagerung des Druckelementes kann aber auch dann vorteilhaft sein, wenn bei einem nicht-vorgeladenen Intraokularlinsenzuführsystem für den Bediener das Einsetzen der Kassette in das Gehäuse nur schwer gelingen sollte. Wird dann der Weg für das Verlagern des Druckelementes zum Beispiel reduziert, bewirkt dies eine geringere Kraft zum Einsetzen der Kassette in das Gehäuse, woraufhin eine geringere Kraft für das Auslösen der Kristallisation verfügbar ist. Sofern die Kristallisation ausgelöst wird, ist ein derart verlagerbares Druckelement immer noch vorteilhaft.

Das Heizelement kann gemäß einer Ausführungsform nur mit der Kassette verbunden sein. Bevorzugt bildet eine einzelne Oberfläche des Heizelementes eine Auflagefläche für die zu erwärmende Intraokularlinse. Die Oberfläche des Heizelementes kann plan oder gewölbt, zum Beispiel konkav, ausgeführt sein. Eine konkave Oberfläche ist vorteilhaft, da somit mehr Wärme in einen Innenbereich der Kassette und damit zu einer in der Kassette aufgenommenen Intraokularlinse gerichtet werden kann. Besonders bevorzugt besitzt das Heizelement benachbart zu der Auflagefläche von dieser abstehende Oberflächen, welche ebenfalls Wärme abstrahlen können. Damit kann eine noch gleichmäßigere Erwärmung des Innenbereiches der Kassette und der darin aufgenommenen Intraokularlinse erreicht werden. Vorteilhaft ist zudem, dass bei einem mit der Kassette verbundenen Heizelement, dessen Oberfläche eine Auflagefläche für die Intraokularlinse bildet, die Kassette in relativ geringen Abmessungen ausgeführt werden kann. Ferner ist es möglich, dass zwischen dem Heizelement und der Intraokularlinse eine Zwischenlage vorhanden ist, welche eine gleichmäßige Wärmeverteilung der vom Heizelement abgegebenen Wärme in Richtung zur Intraokularlinse sicherstellt.

Gemäß einer weiteren Ausführungsform ist das Heizelement nur mit dem Gehäuse verbunden. Das Heizelement ist dann nicht mit der Kassette verbunden. Dies ermöglicht eine Kassette mit sehr geringen Abmessungen, sodass diese wenig Masse besitzt, sich somit sehr schnell durch das Heizelement aufheizen lässt und einfach handhabbar ist.

Wenn das Heizelement mit dem Gehäuse verbunden und die Kassette im Gehäuse eingesetzt ist, befindet sich zwischen der Kassette und dem Heizelement bevorzugt nur ein geringer Abstand im Bereich von 0,001 bis 0,5mm, damit die vom Heizelement freigesetzte Wärmeenergie die Kassette und die darin aufgenommene Intraokularlinse gut erwärmen kann. Besonders bevorzugt befindet sich zwischen dem Heizelement und der Kassette gar kein Abstand, indem ein Flächenkontakt von der Kassette und dem Heizelement besteht. Dadurch wird ein optimaler Wärmeübergang vom Heizelement zur Kassette erreicht.

Durch die Erfindung ist es möglich, einen vorbestimmten Energiebetrag an die Intraokularlinse abzugeben, wobei keine elektrische Energie, keine elektrischen Kontakte und keine Kabel erforderlich sind. Das erfindungsgemäße Intraokularlinsenzuführsystem ist damit genauso gut zu handhaben wie ein nicht heizbares Intraokularlinsenzuführsystem. Dabei ist der technische Aufwand bei dem erfindungsgemäßen Intraokularlinsenzuführsystem sehr gering.

Gemäß einer weiteren Ausführungsform ist das Druckelement ein Vorsprung an einem Flügelelement der Kassette. Eine Kassette kann eine Intraokularlinse in einem noch nicht eingerollten oder gefalteten Zustand enthalten. Wenn ein Bediener die Intraokularlinse in das zu behandelnde Auge einsetzen möchte, kann er die Intraokularlinse durch Schwenken von mindestens einem seitlich an der Kassette angeordneten Flügelelement, siehe zum Beispiel Fig. 14 bis 17 in DE 10 2013 105 185 A1, geringfügig einrollen. Diese Schwenkbewegung erfolgt erst dann, wenn das Einführen der Intraokularlinse in das Auge unmittelbar bevorsteht. Wenn sich gemäß der Ausführungsform ein Vorsprung an einem Flügelelement der Kassette befindet, erfolgt eine Kraftausübung zwischen dem Vorsprung und dem Heizelement und somit der Beginn einer Kristallisation in dem Latentwärmespeicher kurz vor dem stärkeren Einrollen und dem Transport der Intraokularlinse in der Kanüle des Intraokularlinsenzuführsystems. Damit wird erreicht, dass die Wärme automatisch und ohne Betätigung weiterer Vorrichtungselemente im zeitlich günstigen Moment freigesetzt wird, in welchem eine stärkere Elastizität und Formbarkeit der Linse erforderlich ist.

Gemäß einer weiteren Ausführungsform weist das verlagerbare Druckelement einen Stößel auf, mit dem sich auf den Keimbilder eine Druckkraft ausüben lässt. Ein Stößel ermöglicht eine frei einstellbare Kraft auf den Keimbildner. Er erlaubt einen großen Verstellweg, sodass der Stößel in einem eingefahren Zustand nicht stört, wenn die Kassette in das Gehäuse eingesetzt wird. Der Stößel kann zusätzlich zu dem Kolben vorgesehen sein, mit dem die Intraokularlinse durch die Kanüle in das Auge transportiert wird. Es ist jedoch auch möglich, dass der Stößel einstückig mit dem Kolben für den Transport der Intraokularlinse ausgebildet ist. Der Verstellweg des Stößels ist bevorzugt kürzer als der Verstellweg des Kolbens. Der Stößel dient nur dazu, die Kristallisation des Keimbildners auszulösen, sodass sich der Stößel nach diesem Auslösen der Kristallisation des Heizelementes nicht mehr weiter bewegt. Dies kann zum Beispiel dadurch erfolgen, dass sich der Stößel von dem Kolben ausklinkt. Wenn der Stößel keine Bewegung mehr ausführt, bewegt sich dann nur noch der Kolben zum Transport der Intraokularlinse, wenn der Bediener den Kolben betätigt.

In einer weiteren Ausführungsform der Erfindung weist das Gehäuse einen verlagerbaren Gehäuseteil auf. Dies kann vorteilhaft sein, wenn der Gehäuseteil durch einen Bediener auch mit Schutzhandschuhen gut greifbar ist und sich somit auf einfache Weise eine Verlagerung zum Beispiel in Form einer Translationsbewegung erreichen lässt. Dies erleichtert die Handhabbarkeit des erfindungsgemäßen Intraokularlinsenzuführsystems. Das Druckelement ist dann bevorzugt mit dem Gehäuse gekoppelt, wobei durch Verlagerung des Gehäuseteils das Druckelement verlagert wird.

Bevorzugt weist das Heizelement mehrere Keimbildner auf. Wird auf diese Keimbildner jeweils eine Kraft ausgeübt, ist es möglich, an mehreren Stellen im Latentwärmespeicher eine Kristallisation auszulösen. Damit wird an mehreren Stellen eine Wärme freigesetzt, sodass eine bessere Erwärmung der Kassette und einer darin aufgenommenen Intraokularlinse erreichbar ist. Bevorzugt sind diese Keimbildner im Heizelement gleichmäßig verteilt angeordnet. Dadurch kann eine gleichmäßige Erwärmung der Intraokularlinse erreicht werden.

In einer weiteren Ausführungsform der Erfindung weist das Intraokularlinsenzuführsystem zwischen dem Gehäuse und dem Heizelement ein Bauelement auf, welches eine größere Wärmereflektion oder eine größere Wärmeisolation besitzt als das Gehäuse. Durch die Kristallisation wird eine Wärmeenergie freigesetzt, die zunächst nicht in eine Richtung gelenkt ist, sondern von den Kristallisationspunkten kugelförmig abstrahlt. Wenn gemäß dieser Ausführungsform ein Bauelement vorgesehen ist, welches eine größere Wärmereflektion oder eine größere Wärmeisolation besitzt als das Gehäuse, kann die Wärme stärker in die Richtung der Kassette und der darin aufgenommenen Intraokularlinse gerichtet werden, wobei relativ wenig Wärme in Gehäusebereiche abstrahlt, die von der Intraokularlinse abgewandt sind.

Bevorzugt weist das Heizelement ein Volumen im Bereich von 150 bis 500 mm³ auf, und der Latentwärmespeicher setzt nach einer Aktivierung des Keimbildners eine Wärmeenergie im Bereich von 50 bis 300 Joule, bevorzugt 50 bis 100 Joule, frei. Dies ermöglicht es, ein Intraokularlinsenzuführsystem zu schaffen, welches ähnliche Abmessungen und eine ähnlich gut Handhabbarkeit wie ein nicht beheizbares Intraokularlinsenzuführsystem besitzt.

Weitere Vorteile und Merkmale der Erfindung werden mit Bezug auf die nachfolgenden Zeichnungen erklärt, in welchen zeigen:
- Figur 1: eine schematische Darstellung einer ersten Ausführungsform des erfindungsge-mäßen Intraokularlinsenzuführsystems mit einer nicht in ein Gehäuse einge-setzten Kassette;
- Figur 2: eine schematische Darstellung der ersten Ausführungsform des erfindungsge-mäßen Intraokularlinsenzuführsystems, wobei die Kassette in das Gehäuse ein-gesetzt ist;
- Figur 3: eine schematische Darstellung einer Kassette für die erste Ausführungsform des erfindungsgemäßen Intraokularlinsenzuführsystems;
- Figur 4: eine schematische Querschnittsansicht des Intraokularlinsenzuführsystems ge-mäß einer zweiten Ausführungsform;
- Figur 5: eine schematische Querschnittsansicht des Intraokularlinsenzuführsystems ge-mäß einer dritten Ausführungsform;
- Figur 6: eine schematische Darstellung einer vierten Ausführungsform des erfindungs-gemäßen Intraokularlinsenzuführsystems mit einem verlagerbaren Stößel; und
- Figur 7: eine schematische Darstellung einer fünften Ausführungsform des erfindungs-gemäßen Intraokularlinsenzuführsystems mit einem verlagerbaren Gehäuseteil.

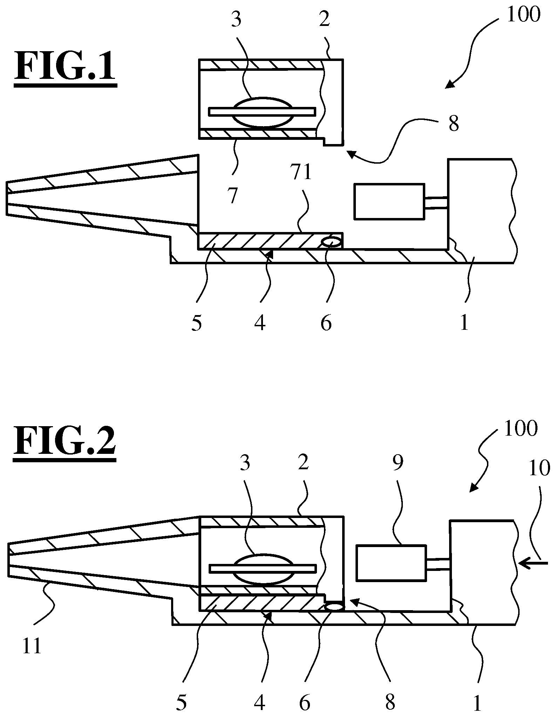

Figur 1 zeigt eine schematische Darstellung einer ersten Ausführungsform des erfindungsgemäßen Intraokularlinsenzuführsystems 100. Das Intraokularlinsenzuführsystem 100 weist ein Gehäuse 1 und eine in das Gehäuse 1 einsetzbare Kassette 2 zur Aufnahme einer Intraokularlinse 3 auf. In der in Figur 1 dargestellten Ausführungsform ist die Kassette 2 noch nicht in dem Gehäuse 1 eingesetzt. Ferner weist das Intraokularlinsenzuführsystem 100 ein Heizelement 4 zum Erwärmen der Kassette 2 und der darin enthaltenen Intraokularlinse 3 auf, wenn die Kassette 2 in das Gehäuse 1 eingesetzt ist. In Figur 1 ist erkennbar, dass das Heizelement 4 mit dem Gehäuse 1 gekoppelt ist. Das Gehäuse 1 kann eine Führungsschiene aufweisen, welche zum Beispiel in vertikaler oder horizontaler Richtung verläuft und geeignet ist, mit einer Nut in der Kassette 2 in Eingriff zu kommen. Das Einsetzen der Kassette 2 in das Gehäuse 1 lässt sich manuell oder mittels einer im Gehäuse 1 vorgesehenen Vorrichtung durchführen. Das Einsetzen der Kassette 2 in das Gehäuse 1 bedeutet, dass die Kassette 2 in der Nähe des Heizelementes 4 positioniert wird.

Das Heizelement 4 weist einen Latentwärmespeicher 5 und einen Keimbildner 6 auf. Ist die Kassette 2 in das Gehäuse 1 eingesetzt, übt ein Druckelement 8, welches bei dieser Ausführungsform mit der Kassette 2 gekoppelt ist, auf den Keimbildner 6 des Heizelementes 4 eine Druckkraft aus. Diese Situation ist in Figur 2 dargestellt. Das Druckelement 8 wirkt auf das Heizelement 4 mit dem darin enthaltenen Keimbildner 6, wobei die Unterseite 7 der Kassette 2 die Oberseite 71 des Heizelementes 4 berührt. Durch das Ausüben einer Druckkraft von dem Druckelement 8 auf den Keimbildner 6 kann in dem Latentwärmespeicher 5 eine Kristallisation ausgelöst werden, wodurch eine Wärmeenergie von dem Latentwärmespeicher 5 zu der Kassette 2 und der darin aufgenommenen Intraokularlinse 3 geleitet wird. Wenn die Intraokularlinse 3 durch die zugeführte Wärmeenergie ausreichend erwärmt ist, kann mittels eines Kolbens 9, welcher in einer Vorschubrichtung entlang des Pfeils 10 in seiner Position veränderbar ist, die Intraokularlinse 3 in Richtung zu einer Kanüle 11 des Intraokularlinsenzuführsystems 100 geführt werden. Die Kanüle 11 ist im Wesentlichen kegelförmig ausgebildet, sodass mit zunehmender Vorwärtsbewegung des Kolbens 9 die Intraokularlinse 3 in dieser Kanüle 11 eingerollt wird. Um wenig Wärmeverluste zu erzielen, ist das Heizelement 4 nur direkt unter der Kassette 2 angeordnet. Es ist aber auch möglich, dass die Kanüle 11 mit beheizt wird.

Der Latentwärmespeicher 5 kann in einem quaderförmig ausgebildeten Heizelement 4 enthalten sein, wobei das Heizelement 4 zum Beispiel die Abmessungen für Länge x Höhe x Breite von 20 mm x 10 mm x 1,25 mm besitzt. Wird als Latentwärmespeicher 5 der Werkstoff Natriumsulfat verwendet, kann bei diesen Abmessungen eine Wärmeenergie im Bereich von etwa 60 bis 70 Joule freigesetzt werden. Wenn ein Wärmeübergangskoeffizient zwischen dem Heizelement 4 und der in das Gehäuse 1 eingesetzten Kassette 2 von etwa 200 W/(m² . K) angenommen wird, kann die Wärmeenergie vom Heizelement 4 zur Kassette 2 und dort zur Intraokularlinse 3 in etwa 1 Minute gelangen, um diese zu erwärmen.

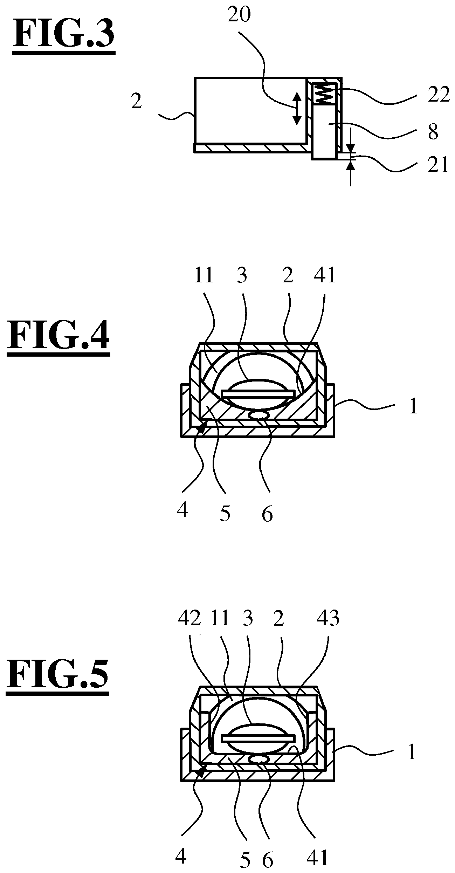

Gemäß der ersten Ausführungsform des erfindungsgemäßen Intraokularlinsenzuführsystems 100 ist die Kassette 2 mit einem verlagerbaren Druckelement 8 versehen, wie dies in Figur 3 dargestellt ist. Das Druckelement 8 lässt sich entlang des Doppelpfeils 20 in vertikaler Richtung in seiner Position verlagern, zum Beispiel mittels eines (nicht in Figur 3 dargestellten) Gewindes, so dass ein veränderbarer Abstand 21 bezüglich der Unterseite 7 der Kassette 2 erreicht wird. Damit können auf einfache Weise Fertigungsungenauigkeiten ausgeglichen werden, so dass immer ein gewünschter Abstand 21 für den Kontakt mit dem Heizelement 4 und dem darin befindlichen Keimbildner 6 erzielt werden kann. Das Druckelement 8 kann durch eine Feder 22 vorgespannt sein, welche sich durch einen Bediener freigeben lässt, wenn die Kristallisation des Keimbildners ausgelöst werden soll. Damit wird nicht nur eine statische Druckkraft, sondern auch ein Stoß auf den Keimbildner 6 ausgelöst, sodass ein Start der Kristallisation sehr zuverlässig erfolgen kann. Allgemein kann ein mechanischer, elektrischer, magnetischer oder chemischer Energiespeicher vorgesehen sein, um das Druckelement 8 zu beschleunigen, sodass dieses mit einer Stoßenergie auf den Keimbildner 6 treffen kann.

Figur 4 zeigt eine Querschnittsansicht des Intraokularlinsenzuführsystems gemäß einer zweiten Ausführungsform mit einer in das Gehäuse 1 eingesetzten Kassette 2, wobei ein Schnitt quer zur Vorschubrichtung 10 dargestellt ist. Die Intraokularlinse 3 liegt direkt auf einer Oberfläche 41 des Heizelementes 4, wobei das Heizelement 4 mit der Kassette 2 verbunden ist. Die Oberfläche 41 ist konkav ausgebildet, sodass eine vom Heizelement 4 abgegebene Wärmeenergie gut in Richtung zur Intraokularlinse 3 orientiert wird.

Figur 5 zeigt eine Querschnittsansicht des Intraokularlinsenzuführsystems gemäß einer dritten Ausführungsform mit einer in das Gehäuse 1 eingesetzten Kassette 2, wobei ein Schnitt quer zur Vorschubrichtung 10 dargestellt ist. Das Heizelement 4, welches hier ebenfalls mit dem Kassette 2 verbunden ist, ist derart ausgeführt, dass es eine plane Auflagefläche 41 für die Intraokularlinse 3 und benachbart zu der Auflagefläche 41 von dieser senkrecht abstehende Oberflächen 42 und 43 aufweist, welche ebenfalls Wärme abstrahlen können. Damit kann eine relativ gleichmäßige Erwärmung des Innenbereiches der Kassette 2 und der darin aufgenommenen Intraokularlinse 3 erreicht werden.

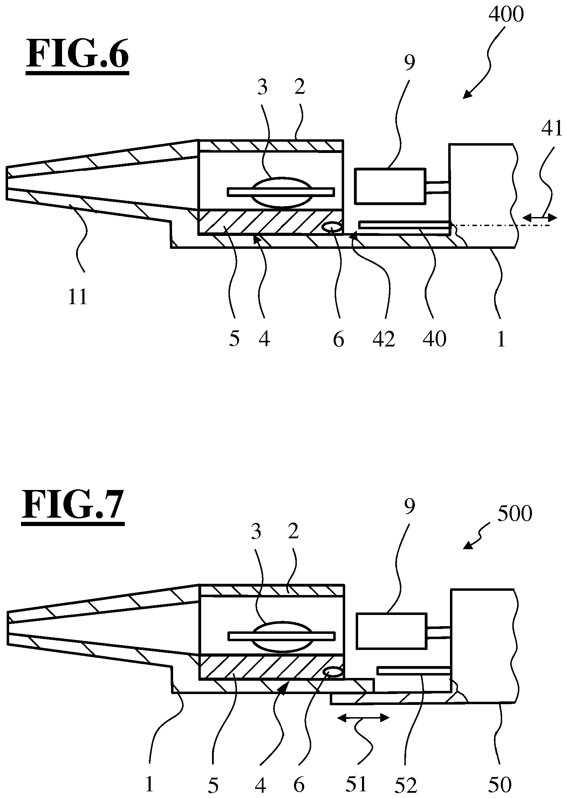

Figur 6 zeigt eine vierte Ausführungsform eines Intraokularlinsenzuführsystems 400, in der die Kassette 2 ohne das Druckelement 8 gemäß der ersten Ausführungsform versehen ist. Bei diesem Intraokularlinsenzuführsystem 400 ist als verlagerbares Druckelement ein Stößel 40 vorgesehen. Der Stößel 40 ist in Richtung des Doppelpfeils 41 beweglich geführt. Durch Verlagerung des Stößels 40 in Richtung zum Heizelement 4 kann mittels einer Spitze 42 des Stößels 40 auf den Keimbilder 6 eine Druckkraft ausgeübt und eine Kristallisation des Keimbildners 6 ausgelöst werden. Das Heizelement 4 ist bei dieser Ausführungsform mit der Kassette 2 verbunden. Es kann jedoch auch mit dem Gehäuse 1 verbunden sein.

Der Stößel 40 kann mit dem Kolben 9 gekoppelt sein oder unabhängig von dem Kolben 9 bewegbar sein. Eine Kopplung von Stößel 40 und Kolben 9 ist vorteilhaft, da somit zum Beispiel nur der Kolben 9 durch einen Bediener zu betätigen ist. Nach Erreichen der erforderlichen Druckkraft für das Auslösen der Kristallisation des Keimbildners 6 kann bei weiterem Vorschub des Kolbens 9 der Stößel 40 ausklinken, sodass nur noch ein Vorschub des Kolbens 9 und der Intraokularlinse 3 erfolgt.

In Figur 7 ist eine fünfte Ausführungsform eines Intraokularlinsenzuführsystems 500 dargestellt. Das Intraokularlinsenzuführsystem 500 weist hier einen in Richtung des Doppelpfeils 51 verlagerbaren Gehäuseteil 50 auf, der ein Bestandteil des Gehäuses 1 ist. Wenn dieser Gehäuseteil 50 ein Druckelement in Form eines Vorsprung 52 zum Beispiel als einen mit dem Gehäuseteil 50 starr gekoppelten Stößel 40 aufweist, kann durch horizontale Verlagerung des Gehäuseteils 50 eine Kraft auf den Keimbildner 6 ausgeübt werden. Anschließend kann in der Vorschubrichtung 10 der Kolben 9 betätigt werden, um die Intraokularlinse 3 zur Kanüle 11 zu befördern. Das Heizelement 4 ist bei dieser Ausführungsform mit der Kassette 2 verbunden. Es kann jedoch auch mit dem Gehäuse 1 verbunden sein.

## Patentansprüche

1. Intraokularlinsenzuführsystem (100; 400; 500) zum Einsetzen einer Intraokularlinse in ein Auge, aufweisend:
- ein Gehäuse (1),
- eine in das Gehäuse (1) einsetzbare Kassette (2) zur Aufnahme einer Intraokularlinse (3),
- ein Heizelement,
**dadurch gekennzeichnet, dass** das Heizelement (4) einen Latentwärmespeicher (5) und einen Keimbildner (6) aufweist,
und das Intraokularlinsenzuführsystem (100; 400; 500) ein Druckelement (8; 40; 52) aufweist, welches mit der Kassette (2) oder dem Gehäuse (1) gekoppelt ist und so verlagerbar ist, dass das Druckelement (8; 40; 52) bei in das Gehäuse (1) eingesetzter Kassette (2) auf den Keimbildner (6) eine Druckkraft ausübt, sodass vom Heizelement (4) Wärmeenergie an die Kassette (2) zugeführt wird.

2. Intraokularlinsenzuführsystem (100; 400; 500) nach Anspruch 1, wobei das Druckelement ein Vorsprung (8) an einem Flügelelement der Kassette (2) ist.

3. Intraokularlinsenzuführsystem (100; 400; 500) nach einem der vorherigen Ansprüche, wobei das Druckelement (8; 40; 52) einen Stößel (40; 52) aufweist.

4. Intraokularlinsenzuführsystem (100; 400; 500) nach einem der vorherigen Ansprüche, wobei das Gehäuse (1) einen verlagerbaren Gehäuseteil (50) aufweist.

5. Intraokularlinsenzuführsystem (100; 400; 500) nach einem der vorherigen Ansprüche, wobei das Heizelement (4) mehrere Keimbildner (6) aufweist.

6. Intraokularlinsenzuführsystem (100; 400; 500) nach einem der vorherigen Ansprüche, welches zwischen dem Gehäuse (1) und dem Heizelement (4) ein Bauelement aufweist, welches eine größere Wärmereflektion oder eine größere Wärmeisolation besitzt als das Gehäuse (1).

7. Intraokularlinsenzuführsystem (100; 400; 500) nach einem der vorherigen Ansprüche, wobei das Heizelement (4) ein Volumen im Bereich von 150 bis 500 mm³ aufweist und der Latentwärmespeicher (5) nach einer Aktivierung des Keimbildners (6) eine Wärmeenergie im Bereich von 50 bis 300 Joule freisetzt.

## Claims

1. Intraocular lens supply system (100; 400; 500) for inserting an intraocular lens into an eye, having:
- a housing (1),
- a cartridge (2) that is insertable into the housing (1), for receiving an intraocular lens (3),
- a heating element (4),
**characterized in that** the heating element (4) has a latent heat store (5) and a nucleation agent (6), and the intraocular lens supply system (100; 400; 500) has a pressure element (8; 40; 52) that is coupled to the cartridge (2) or the housing (1) and displaceable in such a way that the pressure element (8; 40; 52) exerts a compressive force on the nucleation agent (6) when the cartridge (2) is inserted into the housing (1) such that thermal energy is supplied to the cartridge (2) from the heating element (4).

2. Intraocular lens supply system (100; 400; 500) according to Claim 1, wherein the pressure element is a projection (8) at a leaf element of the cartridge (2).

3. Intraocular lens supply system (100; 400; 500) according to either of the preceding claims, wherein the pressure element (8; 40; 52) has a punch (40; 52) .

4. Intraocular lens supply system (100; 400; 500) according to any one of the preceding claims, wherein the housing (1) has a displaceable housing part (50).

5. Intraocular lens supply system (100; 400; 500) according to any one of the preceding claims, wherein the heating element (4) has a plurality of nucleation agents (6).

6. Intraocular lens supply system (100; 400; 500) according to any one of the preceding claims, having a component between the housing (1) and the heating element (4), said component having a greater heat reflection or a greater heat insulation than the housing (1).

7. Intraocular lens supply system (100; 400; 500) according to any one of the preceding claims, wherein the heating element (4) has a volume in the range of 150 to 500 mm³ and the latent heat store (5) releases thermal energy in the range of 50 to 300 joule after activation of the nucleation agent (6) .

## Revendications

1. Système d'amenée de lentille intraoculaire (100 ; 400 ; 500) destiné à insérer une lentille intraoculaire dans un œil, ledit système comprenant :
- un boîtier (1),
- une cassette (2) pouvant être insérée dans le boîtier (1) et destinée à recevoir une lentille intraoculaire (3),
- un élément chauffant (4),
**caractérisé en ce que** l'élément chauffant (4) comporte un accumulateur de chaleur latente (5) et un agent de germination (6),
et le système d'amenée de lentille intraoculaire (100 ; 400 ; 500) comporte un élément de pression (8 ; 40 ; 52) qui est accouplé à la cassette (2) ou au boîtier (1) et qui peut être déplacé de telle manière que, lorsque la cassette (2) est insérée dans le boîtier (1), l'élément de pression (8 ; 40 ; 52) exerce une force de pression sur l'agent de germination (6) de sorte que l'élément chauffant (4) fournisse de l'énergie thermique à la cassette (2).

2. Système d'amenée de lentille intraoculaire (100 ; 400 ; 500) selon la revendication 1, l'élément de pression étant une saillie (8) sur un élément formant ailette de la cassette (2).

3. Système d'amenée de lentille intraoculaire (100 ; 400 ; 500) selon l'une des revendications précédentes, l'élément de pression (8 ; 40 ; 52) comportant un piston (40 ; 52).

4. Système d'amenée de lentille intraoculaire (100 ; 400 ; 500) selon l'une des revendications précédentes, le boîtier (1) comportant une partie de boîtier déplaçable (50).

5. Système d'amenée de lentille intraoculaire (100 ; 400 ; 500) selon l'une des revendications précédentes, l'élément chauffant (4) comportant une pluralité d'agents de germination (6).

6. Système d'amenée de lentille intraoculaire (100 ; 400 ; 500) selon l'une des revendications précédentes, lequel comporte, entre le boîtier (1) et l'élément chauffant (4), un composant dont la réflexion thermique ou l'isolation thermique est supérieure à celle du boîtier (1).

7. Système d'amenée de lentille intraoculaire (100 ; 400 ; 500) selon l'une des revendications précédentes, l'élément chauffant (4) ayant un volume compris entre 150 et 500 mm³ et l'accumulateur de chaleur latente (5) libérant une énergie thermique dans la plage de 50 à 300 joules après activation de l'agent de germination (6) .
